# EUROPEAN PATENT SPECIFICATION

(11) **EP 1 569 688 B1**
(45) Date of publication and mention of the grant of the patent: **10.04.2013**
(21) Application number: 03789540.6
(22) Date of filing: 10.12.2003
(51) Int. Cl.: C07K 16/28

(54) **VACCINE COMPRISING AN ANTIGEN CONJUGATED TO LOW VALENCY ANTI-CD40 ANTIBODIES**
IMPFSTOFF ENTHALTEND EIN ANTIGEN WELCHES AN OLIGOVALENTE ANTI-CD40 ANTIKÖRPER KONJUGIERT IST
VACCIN CONTENANT UN ANTIGENE CONJUGUE A DES ANTICORPS ANTI-CD40& xA;. DE FAIBLE VALENCE

(30) Priority: 11.12.2002 GB 0228796
(43) Date of publication of application: 07.09.2005
(73) Proprietor: Adjuvantix Limited, Sheffield S10 2TN (GB)
(72) Inventor: HEATH, Andrew, Adjuvantix Limited, Sheffield S10 2TN (GB); LAING, Peter, Willingham, Cambridge CB24 5HG (GB)
(74) Representative: Docherty, Robert Charles
(86) International application number: PCT/GB2003/005389
(87) International publication number: WO 2004/052396

(56) References cited:
- WO-A-01/26608
- US-A1- 2002 086 026
- US-A1- 2002 098 184
- CARLRING JENNIFER ET AL: "Anti-CD28 has a potent adjuvant effect on the antibody response to soluble antigens mediated through CTLA-4 by-pass." EUROPEAN JOURNAL OF IMMUNOLOGY, vol. 33, no. 1, January 2003 (2003-01), pages 135-142, XP002274855 ISSN: 0014-2980 (ISSN print)
- BARR TOM A ET AL: "A potent adjuvant effect of CD40 antibody attached to antigen." IMMUNOLOGY, vol. 109, no. 1, May 2003 (2003-05), pages 87-92, XP002274856 ISSN: 0019-2805
- XIANG R ET AL: "A dual-function DNA vaccine encoding carcinoembryonic antigen and CD40 ligand trimer induces T cell-mediated protective immunity against colon cancer in carcinoembryonic antigen-transgenic mice" JOURNAL OF IMMUNOLOGY, THE WILLIAMS AND WILKINS CO. BALTIMORE, US, vol. 167, no. 8, 15 October 2001 (2001-10-15), pages 4560-4565, XP002247590 ISSN: 0022-1767
- FANSLOW W C ET AL: "Structural characteristics of CD40 ligand that determine biological function" SEMINARS IN IMMUNOLOGY, W.B. SAUNDERS COMPANY, PA, US, vol. 6, no. 5, October 1994 (1994-10), pages 267-278, XP002264604 ISSN: 1044-5323

## Description

The invention relates to a conjugate comprising an antibody and antigen wherein said conjugate has low antibody valency and including methods to prepare said conjugate.

The immune system is made up of lymphocytes which are able to recognise specific antigens. B lymphocytes recognise antigens in their native conformation through surface immunoglobulin receptors, and T lymphocytes recognise protein antigens that are presented as peptides along with self molecules known as major histocompatibility antigen (MHC), or human leukocyte antigen (HLA) in humans, on the surface of antigen presenting cells. Antigen presenting cells occur in different forms and may be distinguished into 'classical' antigen presenting cells, exemplified by macrophages and dendritic cells, and 'non-classical' antigen presenting cells, which includes B lymphocytes. T lymphocytes may be further subdivided into "cytotoxic T lymphocytes", which are able to kill virally infected target cells, and "T helper" lymphocytes. T helper lymphocytes have a regulatory function and are able to help B lymphocytes to produce specific antibody, or to help macrophages to kill intracellular pathogens.

Antibodies may exist in several forms, for example there are the main classes: IgM, IgG, IgA, IgD and IgE, each with differing 'effector' functions whereby the effect of the antibody is determined. Effector functions include complement fixation, (resulting in the stimulation of inflammatory responses) which can be activated upon the formation of immune complexes of antigen and antibody by IgM, IgA and IgG. Another example of an effector function is the triggering of mast cells by antigen, which is brought about by the cross-linking of surface IgE on mast cells, tethered there by occupancy of the high-affinity receptor for IgE FcR-epsilon-I (a receptor for the Fc region of IgE). For some of the antibody classes there are subclasses, (e.g. IgG in man is composed of four different subclasses known as IgGI, IgG2, IgG3 and IgG4). The IgG subclasses differ markedly in abundance and in their effector functions.

One of the most important developments in the history of medicine is the advent of vaccines which are used to protect against a wide variety of infectious diseases. There are also vaccines in development for the treatment of various non-infectious diseases such as autoimmune and neurodegenerative diseases and various cancers. Many vaccines are produced by inactivated or attenuated pathogens which are injected into an individual. The immunised individual responds by producing both a humoral (antibody) and cellular (cytolytic T cells, CTL's) response. For example, some influenza vaccines are made by inactivating the virus by chemical treatment with formaldehyde, likewise the Salk polio vaccine comprises whole virus inactivated with propionolactone For many pathogens (particularly bacteria), chemical or heat inactivation, while it may give rise to vaccine immunogens that confer protective immunity, also gives rise to side effects such as fever and injection site reactions. In the case of bacteria, inactivated organisms tend to be so toxic that side effects have limited the application of such crude vaccine immunogens (e.g. the cellular pertussis vaccine). Many modem vaccines are therefore made from protective antigens of the pathogen, separated by purification or molecular cloning from the materials that give rise to side-effects. These latter vaccines are known as 'subunit vaccines'.

The development of subunit vaccines (e.g. vaccines in which the immunogen is a purified protein) has been the focus of considerable research in recent years. The emergence of new pathogens (such as HIV and group-B streptococcus) and the growth of antibiotic resistance have created a need to develop new vaccines and to identify further candidate molecules useful in the development of subunit vaccines. Likewise the discovery of novel vaccine antigens from genomic and proteomic studies is enabling the development of new subunit vaccine candidates, particularly against bacterial pathogens and cancers. However, although subunit vaccines tend to avoid the side effects of killed or attenuated pathogen vaccines, their 'pure' status has separated from the 'danger signals' that are often associated with whole organism vaccines, and subunit vaccines do not always have adequate immunogenicity. Many candidate subunit vaccines have failed in clinical trials in recent years, that might otherwise have succeeded were a suitable adjuvant available to enhance the immune response to the purified antigen. An adjuvant is a substance or procedure which augments specific immune responses to antigens by modulating the activity of immune cells.

We describe an adjuvant with improved efficacy. An adjuvant is a substance or procedure which augments specific immune responses to antigens by modulating the activity of immune cells. Examples of adjuvants include, by example only, Freunds adjuvant, muramyl dipeptides, liposomes. WO97/38711, US02/0136722 and US 02/0086026 discloses, amongst other things, CD28:antigen and CD40: antigen conjugates which act as adjuvants and result in enhanced immune responses directed to the antigen part of the conjugate.

CD28/CD40:antigen conjugates can be produced in a number of ways, and utilising a number of possible cross-linkers. A number of cross-linkers and cross-linking methods are described in the catalogues of the Pierce Chemical Company Inc., and Molecular Probes Inc. Preferred methods of conjugation utilise so-called heterobifunctional cross-linkers, which have different functional groups at each end of the molecule, and thus their use can prevent direct antigen-antigen cross linking, or antibody-antibody cross-linking. However despite the advantages of these cross-linkers, in many cases conjugates can still be formed which contain more than one antibody molecule, and more than one antigen molecule.

For example, if sulfo-SMCC and SATA are used as cross-linkers, one of the conjugate components is first maleimated using sulfoSMCC and the other has sulfhydryl groups attached using SATA. Both the maleimation and the sulfhydryl modification are on primary amines, of which there may be several on both the antibody and the antigen (amino-terminal residues (4 on each Ig molecule), and any lysine residues). It is possible therefore for any antigen to be attached to more than one antibody molecule, and for any antibody molecule to be attached to more than one antigen molecule. In this way large, covalently linked, complexes of antibody and antigen can be formed. The complexes formed during conjugation can be characterised using a number of different parameters:
i) overall size of the conjugate (molecular weight);
ii) ratio of antibody to antigen (weight:weight);
iii) ratio of antibody to antigen (mole: mole);
iv) mean number of antibody molecules in the conjugate; and
v) mean number of antigen molecules in the conjugate.

For any one antigen of known molecular weight, all of these parameters can be derived from (iv) and (v). However as antigens vary in size, the relationships between these values will vary, and thus the optimal forms of conjugates will vary between small (peptides), medium (proteins) and large (polysaccharide) antigens.

From *in vitro* experiments it is known that signalling through both CD40 and CD28 is enhanced by increasing the valency of the interaction. Thus, in order to achieve optimal B or T cell proliferation, anti-CD40 or anti-CD28 are adhered to the plastic of tissue culture plates prior to the addition of the cells, or are cross-linked through the use of anti-Fc antibodies adhered to the plastic, or even through the use of Fc receptor expressing cell lines, such as CD32 expressing L929 cells which are themselves adhered to the tissue culture plastic (Banchereau et al Science 1991 252 70-72.). Surprisingly, we find that , unlike proliferation induction *in vitro,* the adjuvant effects of the antibodies are not enhanced by increased multivalency. Indeed, the adjuvant effects are diminished when either of the antibodies are in a multivalent state.

According to an aspect of the invention there is provided an adjuvant comprising an isolated conjugate of a CD40 antibody and at least one tumour antigen wherein said conjugate consists of an oligomeric complex wherein the antibody valency of the complex consists of one to two antibody molecules per complex.

The formation of a "conjugate" is by any means which results in a conjugation crosslinking or association of antigen with antibody.

According to a further aspect of the invention there is provided a vaccine composition comprising a conjugate according to the invention.

In a preferred embodiment of the invention said composition further comprises a carrier.

In a further preferred embodiment of the invention said composition further comprises a second adjuvant.

The term carrier are construed in the following manner. A carrier is an immunogenic molecule which, when bound to a second molecule augments immune responses to the latter. Some antigens are not intrinsically immunogenic (i.e. not immunogenic in their own right) yet may be capable of generating antibody responses when associated with a foreign protein molecule such as keyhole-limpet haemocyanin or tetanus toxoid. Such antigens contain B-cell epitopes but no T cell epitopes. The protein moiety of such a conjugate (the "carrier" protein) provides T-cell epitopes which stimulate helper T-cells that in turn stimulate antigen-specific B-cells to differentiate into plasma cells and produce antibody against the antigen. Helper T-cells can also stimulate other immune cells such as cytotoxic T-cells, and a carrier can fulfil an analogous role in generating cell-mediated immunity as well as antibodies. Certain antigens which lack T-cell epitopes, such as polymers with a repeating B-cell epitope (e.g. bacterial polysaccharides), are intrinsically immunogenic to a limited extent. These are known as T-independent antigens. Such antigens benefit from association with a carrier such as tetanus toxoid, under which circumstance they elicit much stronger antibody responses. Carrier conjugation of bacterial polysaccharides is used to produce a number of 'conjugate vaccines' against bacterial infections such as *Haemophilus influenzae* (Hib) and group-C meningococci.

In a further preferred embodiment of the invention said antigen is a tumour specific antigen or a tumour associated antigen. Preferably said antigen is selected from the group: a ganglioside antigen a hormone or hormone receptor, for example N-methyl-D aspartate receptor, or part thereof.

A preferred route of administration is intradermal, subcutaneous, intramuscular or intranasal, however the immunisation method is not restricted to a particular mode of administration.

A chimeric antibody is produced by recombinant methods to contain the variable region of an antibody with an invariant or constant region of a human antibody.

A humanised antibody is produced by recombinant methods to combine the complementarity determining regions (CDRs) of an antibody with both the constant (C) regions and the framework regions from the variable (V) regions of a human antibody.

Chimeric antibodies are recombinant antibodies in which all of the V-regions of a mouse or rat antibody are combined with human antibody C-regions. Humanised antibodies are recombinant hybrid antibodies which fuse the complimentarity determining regions from a rodent antibody V-region with the framework regions from the human antibody V-regions. The C-regions from the human antibody are also used. The complimentarity determining regions (CDRs) are the regions within the N-terminal domain of both the heavy and light chain of the antibody to where the majority of the variation of the V-region is restricted. These regions form loops at the surface of the antibody molecule. These loops provide the binding surface between the antibody and antigen.

Antibodies from non-human animals provoke an immune response to the foreign antibody and its removal from the circulation. Both chimeric and humanised antibodies have reduced antigenicity when injected to a human subject because there is a reduced amount of rodent (i.e. foreign) antibody within the recombinant hybrid antibody, while the human antibody regions do not ellicit an immune response. This results in a weaker immune response and a decrease in the clearance of the antibody. This is clearly desirable when using therapeutic antibodies in the treatment of human diseases. Humanised antibodies are designed to have less "foreign" antibody regions and are therefore thought to be less immunogenic than chimeric antibodies.

It is also possible to create single variable regions, so called single chain antibody variable region fragments (scFv's). If a hybridoma exists for a specific monoclonal antibody it is well within the knowledge of the skilled person to isolate scFv's from mRNA extracted from said hybridoma via RT PCR. Alternatively, phage display screening can be undertaken to identify clones expressing scFv's. Alternatively said fragments are "domain antibody fragments". Domain antibodies are the smallest binding part of an antibody (approximately 13kDa). Examples of this technology is disclosed in US6, 248, 516, US6, 291, 158, US6,127, 197 and EP0368684.

According to a further aspect of the disclosure there is provided a method to crosslink an antibody, wherein said antibody is capable of binding a CD40 receptor polypeptide and at least one antigen characterised in that reaction conditions are provided which select for conjugates with low antibody valency.

According to a yet further aspect of the disclosure there is provided a method to prepare a conjugate according to the invention comprising fractionation of a conjugation reaction mixture.

In a preferred method of the disclosure said fractionation comprises the following steps:
i) providing a reaction mixture consisting of a heterogeneous crosslinked antibody: antigen conjugate complex;
ii) separating the reaction mixture into fractions containing conjugates of defined size; and optionally
iii) isolating conjugates with a desired antibody valency.

In a preferred method of the disclosure said fraction contains a conjugate complex with an antibody valency of about on average five antibody molecules per complex.

In a further preferred embodiment of the disclosure said fraction contains a complex of two antibody molecules, preferably said conjugate is a single antibody linked to at least one antigen.

In a preferred method of the disclosure said method is selected from the group consisting of: a size exclusion chromatographic method; an affinity chromatographic method; a differential precipitation method.

An embodiment of the invention will now be provided by example only and with reference to the following figures:
Figure 1 illustrates the effect of anti-CD40 antibody valency on primary anti-rat immune response; and
Figure 2 illustrates the effect of increasing antibody valency on immune responses using an anti-CD40 antibody.

### Materials and Methods

The antibody valency of the conjugates can be varied in a very large number of ways, indeed there are a large number of possible ways to perform the conjugations which will be known to those skilled in the art. The following are included by way of example only.

### Alteration of the degree of derivatisation of the antigen.

One means of cross-linlting antigens with antibodies is to maleimate the antigen, for instance using sulfo-SMCC, and subsequently to react the maleimated antigen with a thiolated antibody (antibody can be thiolated using SATA or SPDP.

The degree of maleimation of the antigen can be altered by changing the relative concentrations of sulfo-SMCC (sulfo-succinimIdyl 4-(N-maleimidomethyl) cyclohexane-1 carboxylate) and antigen in the reaction.

Another method of producing conjugates allows an accurate measurement of the degree of derivatisation of the antigen to be determined. The cross-linker SPDP (succinimidyl 3-(2-pyridyldithio)-propionate) can be used to thiolate the antigen. SPDP reacts at pH 7-9 with an amine containing antigen, yielding a mixed disulfide. Subsequently, upon reduction with dithiothreitol, a 2-pyridinethione chromophore is released and a sulfhydryl group remains on the protein. From the amount of chromophore released (as determined by absorbance) it is possible to calculate the mean ratio of derivatisation on the antigen. Of course if this ratio were, for example, 3 sulfhydryl groups per antigen molecule, the subsequent conjugates with sulfo-SMCC maleimated antibody could not possibly have an antibody valency greater than 3. Thus control of the degree of derivatisation and therefore the mean number of thiol residues per antigen molecule could limit the antibody valency of the conjugates.

Another means of controlling valency would be to alter the number of reactive groups present in the antigen. Thus, for instance SPDP or SATA (among other cross-linkers) might be used to add sulfhydryl groups to the antigen. These cross-linkers both react with primary amines, therefore reaction can be with the amino-terminal residue, or with lysine residues elsewhere in the protein. It is possible to remove some lysine residues from a recombinant antigen by site-directed mutagenesis. It would of course be possible to remove lysine residues from a peptide by altering the synthesis. Of course if the antigenic sequences were being altered it would be important to ascertain that important epitopes were not being removed by this procedure.

### Alteration of Antigen: antibody ratios in the reaction of the derivatised proteins

The relative ratios of derivatised antigen, and antibody in the reaction mix can be altered, and will have effects on the kinds of conjugate formed which will also be dependent upon the degree of derivatisation and the relative sizes of the two components.

### Purification of conjugates of different sizes

The conjugates produced can be purified by size fractionation. For example conjugates of glycoprotein D and antibody of between 200kDa and 400kDa could be separated from larger conjugates by gel filtration. Such conjugates could contain no more than two antibody molecules per conjugate (of approximately 150kDa each).

An alternative method for purification of lower molecular weight conjugates would be to use sequential addition of polyethylene glycol (PEG) to the conjugate mixture. Relatively low concentrations of PEG would be required to precipitate large conjugates, while increasing concentrations will precipitate conjugates of decreasing size. An alternative to PEG would be a salt such as Ammonium sulphate. Again, increasing concentrations of Ammonium sulfate will lead to the precipitation of gradually smaller proteins/conjugates. PEG of Ammonium sulfate can be subsequently removed from the re-dissolved precipitates by dialysis.

An alternative method to select conjugates of low valency rather than small size, would be to deplete high valency conjugates by affinity chromatography on SepharoseCL4B bearing Fc-gamma-receptor-IIb extracellular domain. Only high valency conjugates will stick to the column, or alternatively under isocratic conditions of 0.15M NaCl pH 7.4 10mM Na Po4 buffer, the species will elute in order of valency, i.e. low valency first (unadsorbed or weakly absorbed occurring in the 'void volume' of the column or soon thereafter.

Sizes of the purified conjugates can be assessed by gel filtration against known standards, or in some circumstances by polyacrylamide gel electrophoresis. Activity of the conjugates must then also be determined, most importantly regarding retention of antibody binding to either CD40 or CD28, and retention of antigencity of the antigen indicating that epitopes are intact. One method to verify these tow activities would be to use Flow cytometric staining of CD40 or CD28 expressing cells. Conjugates are added to the cells in PBS and incubated for 30min on ice. Cells are then washed and an antibody (either monoclonal or polyclonal) against the antigen added for 30min on ice. The antibody is then detected using a fluorescently labelled second antibody. Only conjugates with antibody binding (to CD40 or CD28) and antigenic epitopes still intact will give positive staining, and will be ready for assessment of immunogenicity.

### Phage PEG precipitation/purification

It is known that PEG at 15% will precipitate free IgG from serum. Therefore a lower concentration would be appropriate for complexes such as used to preciptitate immune complexes, or larger molecules such as phage virons below.
1. add 30 ml of phage stock to SS-34 Oakridge tube.
2. add 7.5 ml 20 % PEG-8000/2.5 M NaCl.
3. incubate on ice for 30 minutes or longer.
4. spin down phage @ 11K for 20 minutes.
5. respin 2-3x to remove all of PEG solution (using a micro-pipet tip facilitates removal of all solution).
6. resuspend phage in STE (500-1000 ul).
7. transfer to eppendorf and spin @ 14K for 10 minutes.
8. transfer supernatant to new eppendorf and label.
9. titer phage.
STE: for 100 ml add 1 ml 1M Tris (pH 8), 0.2 ml 0.5 M EDTA (pH 8), 2 ml 5 M NaCl. Autoclave.
PEG: for 100 ml add 20 gm PEG-8000 and 14.6 gm NaCl, filter sterilize.

### EXAMPLES 1

Rat (IgG2a) anti-mouse CD40 induces a strongly enhanced immune response in mice against rat IgG2a in comparison with control rat IgG2a. In order to assess the effects of anti-CD40 valency on the adjuvant effect, immunogens of different CD40 antibody valencies were produced as follows.
a. Anti-CD40 antibody or isotype control antibody alone was used as immunogen, valency of one CD40 antibody per "conjugate" (Monomeric)
b. Anti-CD40 or isotype control antibodies were cross linked with anti-rat Ig antibody, to give a valency of two CD40 antibodies per conjugate (dimeric)
c. Anti-CD40 or isotype control antibodies were cross-linked with biotinylated anti-rat Ig, and avidin, to give a multimeric conjugate (multimeric)

In order to ensure that each mouse was immunised with the equivalent mixture of antigens, control proteins were added into the immunogens, such that the immunogens comprised of the following:
a) 10ug CD40 or isotype control mAb, 10ug mouse IgG and 5ug avidin
b) 10ug CD40 or isotype control, mab, 10ug mouse anti rat IgG and 5ug avidin
c) 10ug CD40 or isotype contro, mab, 10ug biotinylated mouse anti rat IgG and 5ug avidin.

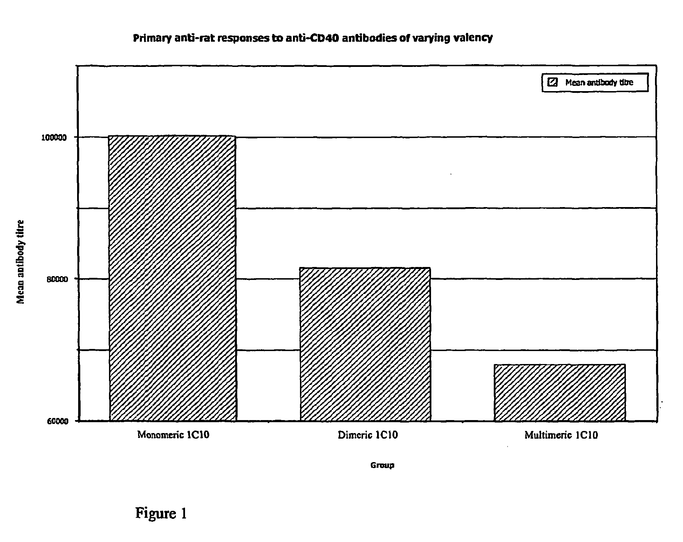

Groups of 5 BALB/c female mice were immunised intraperitoneally, and 10 days later were bled and serum assayed for anti-rat IgG2a responses by ELISA as described previously (Barr et al. Immunology 109 87-91, 2003). Briefly, 96 well ELISA plates were coated overnight with rat IgG2a (GL117) at 10ug/ml in PBS at 4oC. The following day plates were blocked with 1% fish gelatin in PBS and washed with PBS/0.05% Tween. Serial dilutions of sera were made, and after incubation for 1h at room temperature, and washing, conjugate (horse radish peroxidase labelled goat anti-mouse immunoglobulins, multiadsorbed (Sigma) was added to wells, and the plates incubated for a further hour. Plates were then washed again, and incubated with substrate (OPD, Sigma) for 15 minutes and read at 490nm. Titres are expressed as the reciprocal of the highest serum dilution at which test serum gave a higher OD then normal mouse serum. Results are shown in Figure 1.

### EXAMPLE 2

Increasing valency of the conjugates reduces the antibody response. Keyhole limpet hemacyanin (Sigma) was derivatised with SPDP (Molecular probes, using protocols provided by Molecular probes) and the sulfhydryl groups deprotected by reduction with dithiothreitol, followed by dialysis. Anti-CD40 or isotype control antibody were meleimated using sulfo-SMCC (Molecular probes, using proteocols provided by Molecular probes), and the derivatised proteins mixed together to form conjugates as follows:
A) 100% anti-CD40 antibody (5mg antibody to 1mg KLH)
B) 10% anti-CD40, 90% isotype control antibody (5mg antibody to 1mg KLH)
C) 1% anti-CD40, 99% isotype control antibody (5mg antibody to 1mg KLH

This protocol was designed to produce conjugates of the same size and antigen content, but with different CD40 antibody valencies.

Groups of 3 BALB/c female mice were immunised intraperitoneally, and 10 days later were bled and serum assayed for anti-KLH responses by ELISA as described previously (Barr et al. Immunology 109 87-91, 2003). Briefly 96 well ELISA plates were coated overnight with KLH at 10ug/ml in PBS at 4oC. The following day plates were blocked with 1% fish gelatin in PBS and washed with PBS/0.05% Tween. Serial dilutions of sera were made, and after incubation for 1h at room temperature, and washing, conjugate (horse radish peroxidase labelled goat anti-mouse immunoglobulins, multiadsorbed (Sigma) was added to wells, and the plates incubated for a further hour. Plates were then washed again, and incubated with substrate (OPD, Sigma) for 15 minutes and read at 490nm. Titres are expressed as the reciprocal of the highest serum dilution at which test serum gave a higher OD then normal mouse serum.

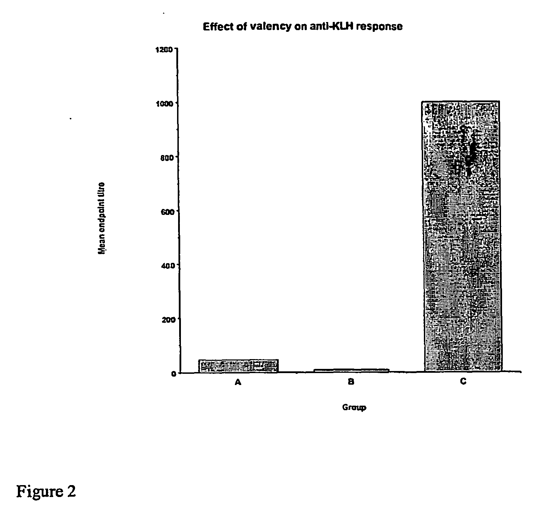

Results are shown in Figure 2. The strongest antibody responses against KLH were produced by mice immunised with the 1% CD40 conjugates. As SPDP derivatisation was estimated to produce 200 reactive sites per KLH molecule, the estimated maximum CD40 antibody valency of the conjugates used in group C was 2 molecules of anti-CD40 per KLH molecule.

## Claims

1. A vaccine composition comprising: an isolated conjugate of a CD40 antibody and at least one tumour specific or tumour associated antigen wherein said conjugate consists of a complex wherein the CD40 antibody and tumour antigen are crosslinked with a cross-linker and the antibody valency of the complex consists of one or two CD40 antibody molecules.

2. A composition according to claim 1, wherein said composition further comprises a carrier.

3. A composition according to claim 2, wherein said composition further comprises a second adjuvant.

4. A composition according to claim 1, wherein said antigen is a ganglioside antigen.

5. A composition according to claim 1, wherein said antigen is a hormone or hormone receptor.

6. A composition according to claim 5, wherein said antigen is the N-methyl-D aspartate receptor, or part thereof.

## Patentansprüche

1. Impfstoffzusammensetzung, umfassend: ein isoliertes Konjugat aus einem CD40-Antikörper und mindestens einem tumorspezifischen oder tumorassoziiertes Antigen, wobei das Konjugat aus einem Komplex besteht, wobei der CD40-Antikörper und das Tumorantigen mit einem Vernetzungsmittel vernetzt sind, und die Antikörper-Wertigkeit des Komplexes aus einem oder zwei CD40-Antikörper-Molekülen zusammengesetzt ist.

2. Zusammensetzung nach Anspruch 1, wobei die Zusammensetzung ferner einen Trägerstoff umfasst.

3. Zusammensetzung nach Anspruch 2, wobei die Zusammensetzung ferner ein zweites Adjuvans umfasst.

4. Zusammensetzung nach Anspruch 1, wobei das Antigen ein Gangliosid-Antigen ist.

5. Zusammensetzung nach Anspruch 1, wobei das Antigen ein Hormon oder Hormon-Rezeptor ist.

6. Zusammensetzung nach Anspruch 5, wobei das Antigen der N-Methyl-D-aspartat-Rezeptor oder ein Teil davon ist.

## Revendications

1. Composition de vaccin comprenant : un conjugué isolé d'un anticorps CD40 et au moins un antigène propre à une tumeur ou associé à une tumeur, dans laquelle ledit conjugué est constitué d'un complexe dans lequel l'anticorps CD40 et l'antigène de tumeur sont réticulés avec un agent de réticulation et où la valence d'anticorps du complexe est constituée d'une ou deux molécules d'anticorps CD40.

2. Composition selon la revendication 1, ladite composition comprenant en outre un véhicule.

3. Composition selon la revendication 2, ladite composition comprenant en outre un deuxième adjuvant.

4. Composition selon la revendication 1, dans laquelle ledit antigène est un antigène ganglioside.

5. Composition selon la revendication 1, dans laquelle ledit antigène est une hormone ou un récepteur d'hormone.

6. Composition selon la revendication 5, dans laquelle ledit antigène est le récepteur de N-méthyl-D aspartate, ou une partie de celui-ci.
